# NEUE EUROPÄISCHE PATENTSCHRIFT

(11) **EP 0 402 508 B2**
(45) Veröffentlichungstag und Bekanntmachung des Hinweises auf die Entscheidung über den Einspruch: **23.10.2002**
(45) Hinweis auf die Patenterteilung: 15.03.1995
(21) Anmeldenummer: 89110885.4
(22) Anmeldetag: 15.06.1989
(51) Int. Cl.: A61B 5/046

(54) **Einrichtung zum Detektieren einer Folge von anormalen Ereignissen in einem elektrischen Signal, insbesondere dem Depolarisationssignal eines Herzens**
Means for detecting a series of abnormal events in an electrical signal, especially the depolarisation signal of a heart
Dispositif pour détecter une série d'événements anormaux dans un signal électrique, notamment dans un signal de dépolarisation du coeur

(43) Veröffentlichungstag der Anmeldung: 19.12.1990
(73) Patentinhaber: St. Jude Medical AB, 175 84 Järfälla (SE)
(72) Erfinder: Andersen, Hans, Dipl.-Ing., S-162 35 Vällinby (SE)
(74) Vertreter: Harrison, Michael Charles

(56) Entgegenhaltungen:
- DE-A- 2 643 907
- US-A- 3 703 900
- US-A- 3 861 387
- US-A- 4 342 318
- US-A- 4 523 595

## Beschreibung

Einrichtung zum Delektieren einer Folge von anormalen Ereignissen in einem elecktrodenphysiologischen Signal, insbesondere dem Depolarisationssignal eines Herzens.

Die Erfindung betrifft eine Einrichtung zum Detektieren einer Folge von anormalen Ereignissen unter einer Vielzahl von normalen Ereignissen in einem elektrophysiologischen Signal. Die Ausführungsformen der Erfindung betreffen ausserdem eine Einrichtung zum Detektieren einer Folge von auf eine Fibrillation hindeutenden Ereignissen unter einer Vielzahl von normalen Ereignissen in dem Depolarisationssignal eines eine Funktionsstörung aufweisenden Herzens, eine Einrichtung zum Detektieren der Fibrillation eines eine Funktionsstörung aufweisenden Herzens sowie eine Einrichtung zur selbsttätigen Defibrillation eines eine Funktionsstörung aufweisenden Herzens.

Das Detektieren einer Folge von anormalen Ereignissen unter einer Vielzahl von normalen Ereignissen in einem elektrophysiologischen Signal spielt z.B. bei der Detektion von Arrhythmien anhand des Depolarisationssignals eines eine Funktionsstörung aufweisenden Herzens eine Rolle. Das Depolarisationssignal eines eine Funktionsstörung aufweisenden Herzens enthält neben auf normale Herztätigkeit hindeutenden Wellenformen beim Auftreten einer Arrhythmie, z.B. Herzflimmern (Fibrillation), Wellenformen, die von den im Falle von normaler Herztätigkeit vorliegenden Wellenformen abweichen. Fasst man die jeweils einem normalen oder anormalen Herzschlag entsprechenden Wellenformen als normales bzw. anormales Ereignis in dem Depolarisationssignal des Herzens auf, stellt eine Arrhythmie eine Folge von anormalen Ereignissen unter einer Vielzahl von normalen Ereignissen dar.

Da im Zusammenhang mit Arrhythmien lebensbedrohliche Situationen für den Patienten auftreten können, ist es wünschenswert, eine Folge von auf eine Arrhythmie hindeutenden Ereignissen unter einer Vielzahl von normalen Ereignissen in dem Depolarisationssignal eines Herzens rasch und zuverlässig detektieren zu können, um etwa erforderliche therapeutische Massnahmen rechtzeitig einleiten zu können. Insbesondere im Zusammenhang mit selbsttätigen Defibrillatoren ist es unerlässlich, eine Folge von auf eine Fibrillation des Herzens hindeutenden Ereignissen unter einer Vielzahl von normalen Ereignissen sicher und zuverlässig detektieren zu können. Nur so ist sichergestellt, dass einerseits das Herz die zur Beendigung der Fibrillation erforderlichen Defibrillationsimpulse auch tatsächlich erhält und andererseits unnötige Defibrillationsimpulse, die katastrophale Folgen nach sich ziehen können, vermieden sind.

In der US-Patentschrift 3,861,387 ist ein Gerät zur Detektion von Herzrhytmusstörungen beschrieben. Dabei wird ein der elektrischen Aktivität des Herzens entsprechendes Signal gewonnen und von diesem Signal die erste Ableitung nach der Zeit gebildet. Für jede normale oder anormale Tätigkeit des Herzens, also für jedes normale oder anormale Ereignis in dem differenzierten Signal, wird die Spitzenamplitude ermittelt und für eine bestimmte Anzahl der zuletzt gemessenen Spitzenamplituden laufend der Mittelwert gebildet. Ausgehend von diesem fortlaufend ermittelten Mittelwert wird ein Schwellwert definiert und für jedes Ereignis in dem differenzierten Signal die Zeitdauer ermittelt, für die sich die Amplitude während der Dauer des Ereignisses oberhalb des Schwellwertes bewegt. Auch bezüglich einer bestimmten Anzahl von jeweils zuletzt gemessenen Zeitdauern wird fortlaufend der Mittelwert gebildet. Für jedes in dem differenzierten Signal aufgetretene Ereignis wird sowohl die Spitzenamplitude als auch die Zeitdauer, für die sich die Amplitude oberhalb des Schwellwertes bewegte, mit dem Mittelwert verglichen. Sobald ein Ereignis bezüglich wenigstens eines Mittelwertes eine Abweichung aufweist, die einen bestimmten Grenzwert überschreitet, wird ein Signal erzeugt, das auf das Vorliegen eines anormalen Ereignisses hindeutet. Der Entscheidung, ob ein anormales oder ein normales Ereignis vorliegt, liegt also kein objektives Kriterium zugrunde. Sie erfolgt vielmehr anhand von Kriterien, nämlich Mittelwerten, die aus dem zu untersuchenden Signal abgeleitet sind. Dies mag noch zu brauchbaren Ergebnissen führen, solange einzelne anormale Ereignisse zu detektieren sind, insbesondere da als anormal detektierte Ereignisse bei der Bildung der Mittelwerte ausser Betracht bleiben. Die Detektion von Folgen von anormalen Ereignissen, die im Falle der US-A-3,861,387 in der Weise erfolgen soll, dass die Abgabe von auf anormale Ereignisse hindeutenden Signalen solange unterbunden wird, bis eine bestimmte Anzahl von anormalen Ereignissen in einem definierten Zeitintervall detektiert wurde, ist jedoch nicht mit ausreichender Zuverlässigkeit möglich. Verändert sich nämlich ein zunächst im wesentlichen nur objektiv normale Ereignisse enthaltendes Signal allmählich, also ohne dass in nennenswertem Umfang Detektionen anormaler Ereignisse auftreten, dahingehend, dass Folgen von Ereignissen oder sämtliche Ereignisse in dem Signal objektiv als anormal einzustufen sind, werden diese Ereignisse dennoch nicht detektiert, da mit der allmählichen Veränderung des Signals eine allmähliche Veränderung der Mittelwerte stattgefunden hat und nun objektiv anormale Ereignisse fälschlicherweise als normale Ereignisse angesehen werden.

In der US-Patentschrift 4 523 595 ist ein Gerät zur Detektion eines Fibrillationszustandes des Herzens beschrieben. Bei diesem Gerät werden Werte, bezüglich typischer Parameter des Herzsignals, mit eingespeicherten Werten dieser Parameter für verschiedene Herzzustände verglichen. Da bei einem Fibrillationszustand das Herzsignal völlig unorganisiert ist, kann dieser Zustand festgestellt werden, wenn keine von den dabei vorliegenden Parameterwerten eine Ähnlichkeit mit den eingespeicherten Werten zeigen. Dieses Gerät benutzt nicht nur die Spitzenamplitude des abgeleiteten Herzsignals, sondern zusätzlich auch noch z.B die Spitzenamplitude des Herzsignals selbst, wobei die Spitzenamplitude eines Ereignisses mit einem Mittelwert der des zu untersuchenden (Herz)signals, verglichen wird. Auch bei diesem Gerät liegt also der Entscheidung, ob ein anormaler Zustand vorliegt, kein objektives Kriterium zugrunde.

Der Erfindung liegt die Aufgabe zugrunde, eine Einrichtung zum Detektieren einer Folge von anormalen Ereignissen in einem elektrophysiologischen Signal, insbesondere dem Depolarisationssignal eines Herzens, so auszubilden, dass die Detektion einer Folge von anormalen Ereignissen in dem Signal unter allen Umständen, insbesondere auch bei allmählichen Änderungen der Erscheinungsform der Ereignisse, mit hoher Zuverlässigkeit gewährleistet ist.

Im Falle der erfindungsgemässen Einrichtung wird also bezüglich der Maximalwerte eines für die jeweils zu detektierenden Ereignisse typischen Signalparameters, es kann sich hierbei z.B. um die Amplitude, die Steilheit (slope) des Signals, dessen Frequenz usw. handeln, eine deren statistischer Verteilung entsprechende Kenngrösse ermittelt. Diese wird mit einem Sollwert der Kenngrösse verglichen, um entscheiden zu können, ob die bei der Bildung der Kenngrösse berücksichtigten Ereignisse eine Folge von anormalen Ereignissen enthalten bzw. selbst Bestandteile einer Folge von anormalen Ereignissen sind. Die Detektion einer Folge von anormalen Ereignissen erfolgt also anhand eines objektiven Kriteriums, da der Sollwert der Kenngrösse in keiner Weise von dem Verlauf des Signals abhängt. Wesentlich ist ausserdem, dass das Signal hinsichtlich desjenigen Signalparameters, dessen Maximalwerte für jedes Ereignis gemessen werden, mit einem definierten Schwellwert verglichen wird, den das Signal sowohl beim Auftreten eines normalen als auch eines anormalen Ereignisses überschreitet. Durch geeignete Wahl des Schwellwertes kann also vermieden werden, dass Störungen oder dergleichen fälschlicherweise als Ereignis angesehen und bei der Ermittlung der der statistischen Verteilung der gemessenen Maximalwerte entsprechenden Kenngrösse berücksichtigt werden. Die Einrichtung gestattet es also, eine Folge von anormalen Ereignissen unter einer Vielzahl von normalen Ereignissen in einem elektrischen Signal mit hoher Zuverlässigkeit zu detektieren. Dabei steigt die Zuverlässigkeit der Detektion mit der Anzahl von Ereignissen, die zur Ermittlung der statistischen Verteilung der gemessenen Maximalwerte entsprechenden Kenngrösse herangezogen werden. Es versteht sich, dass zugleich diejenige Zeit steigt, die von dem Auftreten einer Folge von anormalen Ereignissen bis zu demjenigen Zeitpunkt vergeht, zu dem der Vergleich der ermittelten Kenngrösse mit dem Sollwert der Kenngrösse auf das Vorhandensein einer Folge von anormalen Ereignissen hindeutet. Es muss also ein Kompromiss zwischen der zur Detektion erforderlichen bzw. zulässigen Zeit und der Anzahl der zur Ermittlung der Kenngrösse heranzuziehenden Anzahl von Ereignissen geschlossen werden. Aus den vorstehenden Ausführungen wird klar, dass die erfindungsgemässe Einrichtung zum Detektieren einzelner anormaler Ereignisse unter einer Vielzahl von normalen Ereignissen weniger geeignet ist.

Als der statistischen Verteilung der gemessenen Maximalwerte entsprechende Kenngrösse kommt z.B. der arhithmetische Mittelwert in Frage. Gemäss einer besonders vorteilhaften Ausführungsform der Erfindung wird als der statistischen Verteilung der gemessenen Maximalwerte entsprechende Kenngrösse die Standardabweichung ermittelt. Die Standardabweichung gestattet selbst dann, wenn die gemessenen Maximalwerte der Ereignisse innerhalb gewisser Grenzen streuen, eine exakte Entscheidung darüber, ob eine Folge anormaler Ereignisse vorliegt oder nicht.

Bezüglich der Definition der üblicherweise mit dem Formelzeichen S bezeichneten Standardabweichung wird auf das Buch von Dr.-Ing. W. Walcher, Praktikum der Physik, 3, Auflage, Verlag B.G. Teubner, Stuttgart, 1974, Seiten 24 ff. verwiesen. Analoge oder digitale Schaltungen zur Ermittlung der Standardabweichung sind übrigens aus dem Stand der Technik bekannt.

Nach einer Ausführungsform der Erfindung wird für jedes Ereignis die Differenz zwischen dem Maximalwert und dem Schwellwert des Signalparameters berechnet und jeweils der Ermittlung der statistischen Verteilung der gemessenen Maximalwerte entsprechenden Kenngrösse zugrundegelegt. Die Standardabweichung wird also anhand eines sogenannten Detektionsmariginals, das, falls dies wünschenswert ist, auch als Bruchteil oder Prozentsatz des Schwellwertes ausgedrückt werden kann, ermittelt. Dies ist besonders anschaulich, da die Relationen der Maximalwerte zu dem Schwellwert sofort erkennbar sind.

Die Aufgabe wird durch die Einrichtung gemäss Patentanspruch 1 gelöst.

Die Vorteile dieser Einrichtung ergeben sich aus der vorangegangenen Beschreibung. Die Einrichtung besitzt ausserdem einen einfachen Aufbau, da Mittel zur Messung des Maximalwertes und Mittel zum Ermitteln einer der statistischen Verteilung der gemessenen Maximalwerte entsprechenden Kenngrösse nur bezüglich eines einzigen Signalparameters vorhanden sein müssen, wobei gemäss einer bevorzugten Ausführungsform der Erfindung die Mittel zum Ermitteln einer der statistischen Verteilung der gemessenen Maximalwerte entsprechenden Kenngrösse die Standardabweichungen ermitteln. Für den Fall, dass aus Gründen der Anschaulichkeit bezuglich der einzelnen Ereignisse deren Maximalwerte in Relation zu dem Schwellwert als Detektionsmariginal angegeben werden soll, sind gemäss einer Ausführungsform der Erfindung Mittel zur Bestimmung der Differenz zwischen dem jeweils für ein Ereignis gemessenen Maximalwert und dem Schwellwert des Signalparameters vorgesehen, wobei den Mitteln zum Ermitteln einer statistischen Verteilung der gemessenen Maximalwerte entsprechenden Kenngrösse bezüglich der einzelnen Ereignisse jeweils die Differenzen zugeführt sind.

Eine Einrichtung zum Detektieren einer Folge von auf eine Fibrillation hindeutenden Ereignissen unter einer Vielzahl von normalen Ereignissen in dem Depolarisationssignal eines einer Funktionsstörung aufweisenden Herzens ist im Patentanspruch 4 angegeben.

Als Signalparameter wird also diejenige Zeitdauer herangezogen, für die die Amplitude des Signals jeweils beginnend von einem Zeitpunkt, zu dem die Steilheit des Depolarisationssignals eine definierte Steilheit erreicht, mindestens dem Produkt aus definierter Steilheit und verstrichener Zeit entspricht. Den Schwellwert bildet somit eine Mindestzeitdauer, die das Depolarisationssignal sowohl beim Auftreten eines normalen als auch eines auf eine Fibrillation hindeutenden Ereignisses überschreitet. Als Maximalwert wird die Gesamtzeitdauer gemessen, für die die Amplitude des Depolarisationssignals während des jeweiligen Ereignisses mindestens dem Produkt aus definierter Steilheit und verstrichener Zeit entspricht Als statistische Kenngrösse wird schliesslich die Standardabweichung der Gesamtzeitdauern bzw. von den Gesamtzeitdauern entsprechenden Grössen (Detektionsmarginal) ermittelt.

Es wurde festgestellt, dass das Depolarisationssignal des Herzens im Falle von normalen Herzschlägen (Sinus-Rhythmus) für jedes Ereignis etwa die gleiche Gesamtzeitdauer aufweist, für die die Amplitude des Depolarisationssignals während des jeweiligen Ereignisses mindestens dem Produkt aus definierter Steilheit und verstrichener Zeit entspricht. Entsprechend gering ist die der Standardabweichung der für das jeweils zuletzt aufgetretene und eine definierte Anzahl von fortlaufend unmittelbar vor diesem aufgetretenen Ereignissen gemessene Gesamtzeitdauern entsprechende statistische Kenngrösse. Folgt das Herz nicht mehr dem Sinus-Rhythmus, was z.B. im Falle von Fibrillation der Fall ist, schwanken die für die einzelnen Ereignisse gemessenen Gesamtzeitdauern ganz erheblich, was seinen Niederschlag in einer Vergrösserung der Standardabweichung der Gesamtzeitdauern findet. Mittels der erfindungsgemässen Einrichtung ist also eine zuverlässige Detektion einer Folge von anormalen Ereignissen, denen ein anderer als der Sinus-Rhythmus zugrunde liegt, möglich. Dies bedeutet, dass Folgen von anormalen Ereignissen detektierbar sind, die auf eine Fibrillation des Herzens hindeuten.

Eine Einrichtung zum Detektieren der Fibrillation eines eine Funktionsstörung aufweisenden Herzens ist im Patentanspruch 5 angegeben.

Es wird hier also nicht nur die Standardabweichung der im Zusammenhang mit einer definierten Anzahl von zuletzt aufgetretenen Ereignissen gemessenen Gesamtzeitdauern bzw. eine dieser entsprechende statistische Kenngrösse, sondern zusätzlich die Herzschlagfrequenz, die beim Auftreten von Fibrillationen oberhalb einer bestimmten Grenze liegt, berücksichtigt. Somit ist eine zuverlässige Detektion der Fibrillation des Herzens gewährleistet.

Eine Einrichtung zur selbsttätigen Defibrillation eines eine Funktionsstörung aufweisenden Herzens weist gemäss Patentanspruch 6 zusätzlich Mittel zur Beaufschlagung des Herzens mit Defibrillationsimpulsen auf, die mit den Mitteln zum Erzeugen eines Signales verbunden sind und bei Vorliegen eines Signales das Herz mit wenigstens einem Defibrillationsimpuls beaufschlagen. Da die Mittel zum Erzeugen eines Signals wie erwähnt ein Signal nur abgeben, wenn zwei Bedingungen erfüllt sind, ist die Gefahr, dass das Herz mit Defibrillationsimpulsen beaufschlagt wird, ohne dass eine Fibrillation vorliegt, äusserst gering.

Im Folgenden wird die Erfindung anhand von 6 FIG näher erläutert. Es zeigen:
- FIG 1: in Form eines Blockschaltbildes eine erfindungsgemässe Einrichtung zur Detektion einer Folge von anormalen Ereignissen unter einer Vielzahl von normalen Ereignissen in einem elektrischen Signal
- FIG 2: eine Einrichtung zur selbsttätigen Defibrillation eines eine Funktionsstörung aufweisenden Herzens, welche eine Einrichtung gem. FIG 1 enthält, als Blockschaltbild.
- FIG 3: in detaillierter Darstellung einen Teil der Schaltung der Einrichtung gem. FIG 2,
- FIG 4: das eine Anzahl von aufeinanderfolgenden normalen Ereignissen enthaltende Depolarisationssignal eines Herzens, und
- FIG 5: das eine Folge von anormalen, auf eine Fibrillation hindeutenden Ereignissen enthaltende Depolarisationssignal eines eine Funktionsstörung aufweisenden Herzens.

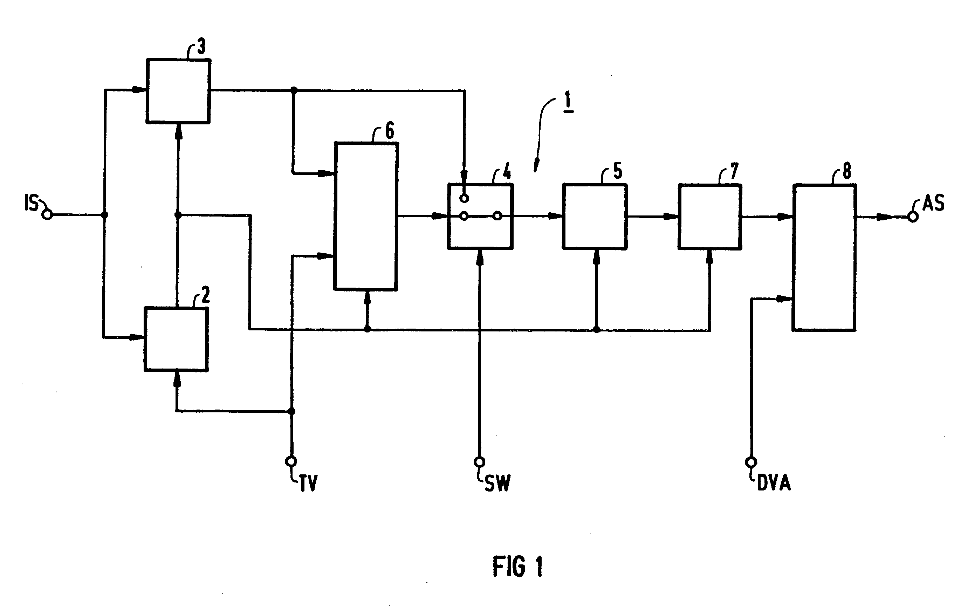

In FIG 1 ist eine insgesamt mit 1 bezeichnete Einrichtung zur Detektion einer Folge von anormalen Ereignissen unter einer Vielzahl von normalen Ereignissen in einem elektrischen Signal IS dargestellt. Gemäss FIG 1 gelangt das Eingangssignal IS zu einer Detektorschaltung (2), die das Signal IS bezüglich eines Signalparameters (z.B. Amplitude und/oder Steilheit) mit einem definierten Schwellwert vergleicht. Im Falle der FIG 1 wird ein dem definierten Schwellwert entsprechendes Signal TV der Detektorschaltung (2) von aussen zugeführt, so dass die Höhe des Schwellwertes, den das Signal IS sowohl beim Auftreten eines normalen als auch eines anormalen Ereignisses überschreitet, durch Verändern des Signales TV programmierbar ist. Die Detektorschaltung (2) kann aber auch so ausgebildet sein, dass ein fester Schwellwert vorhanden ist. Jedesmal, wenn ein Ereignis auftritt, das hinsichtlich des Signalparameters den definierten Schwellwert überschreitet, gibt die Detektorschaltung (2) ein Detektionssignal DS ab.

Das Signal IS gelangt ausserdem zu einem Spitzenwertmesser (3), der auf den gleichen Signalparameter wie die Detektorschaltung (2) anspricht. Durch das Detektionssignal DS, das einem Steuereingang des Spitzenwertmessers (3) zugeführt ist, wird dieser derart aktiviert, dass er jedesmal beim Auftreten eines Ereignisses (Detektionssignal DS) den Maximalwert des besagten Signalparameters misst. Falls der Umschalter (4) die in FIG 1 dargestellte Stellung einnimmt, gelangt der mittels des Spitzenwertmessers (3) für das jeweils aufgetretene Ereignis gemessene Maximalwert zu einem Speicher (5), der derart ausgebildet ist, dass er den für das zuletzt aufgetretene Ereignis gemessenen Maximalwert und die für eine definierte Anzahl von aufeinanderfolgend unmittelbar vor diesem aufgetretenen Ereignissen gemessenen Maximalwerte speichert. Dabei wird jeweils beim Speichern des Maximalwertes des zuletzt aufgetretenen Ereignisses der Maximalwert des "ältesten" Ereignisses gelöscht. Die beschriebenen Vorgänge werden durch das beim Auftreten eines Ereignisses von der Detektorschaltung (2) jeweils abgegebene Detektionssignal DS gesteuert, das dem Speicher (5) zugeführt ist.

Wird der Umschalter (4), es handelt sich hierbei vorzugsweise um einen elektronischen Umschalter, durch Anlegen eines Umschaltsignales SW in seine andere Schaltposition gebracht, ist der Speicher (5) mit dem Ausgang einer Subtrahierschaltung (6) verbunden, die durch das Detektionssignal DS gesteuert für jedes Ereignis die Differenz zwischen dem zugehörigen Maximalwert und dem Schwellwert des Signalparameters bildet. Zu diesem Zweck ist der Subtrahierschaltung (6) ausser dem Ausgangssignal des Spitzenwertmessers (3) das dem Schwellwert entsprechende Signal TV zugeführt.

Je nach Stellung des Umschalters (4) sind also für das zuletzt aufgetretene Ereignis und eine definierte Anzahl unmittelbar vor diesem aufgetretenen Ereignissen entweder die Maximalwerte des Signalparameters oder die Differenzen zwischen diesen Maximalwerten und dem Schwellwert gespeichert, wobei die Differenzen den Detektionsmarginalen für die jeweiligen Ereignisse entsprechen.

Jedesmal beim Auftreten eines neuen Ereignisses werden die in dem Speicher (5) gespeicherten Maximalwerte bzw. Differenzen einschl. des entsprechenden Wertes für das zuletzt aufgetretene Ereignis einer Rechenschaltung (7) zugeführt, die eine der statistischen Verteilung der gemessenen Maximalwerte bzw. der errechneten Differenzen entsprechende Kenngrösse ermittelt, z.B. deren arhithmetischen Mittelwert. Die Rechenschaltung (7) ermittelt also in jedem Fall eine der statistischen Verteilung der gemessenen Maximalwerte entsprechende Kenngrösse, unabhängig davon, ob diese auf Grundlage der gemessenen Maximalwerte selbst oder den aus diesen und dem Schwellwert gebildeten Differenzen bzw. Detektionsmarginalen errechnet wird.

Ein der errechneten statistischen Kenngrösse entsprechendes Signal wird einem Vergleicher (8) zugeführt, der dieses mit einem Sollwert der statistischen Kenngrösse entsprechenden Signal DVA vergleicht, das seinem anderen Eingang zugeführt ist. Der Sollwert bzw. das diesem entsprechende Signal DVA ist derart gewählt, dass dann, wenn das elektrische Signal IS im wesentlichen ausschliesslich normale Ereignisse enthält, kein Ausgangssignal AS des Vergleichers (8) vorhanden ist. Wenn jedoch das Signal IS eine Folge von anormalen Ereignissen bzw. ausschliesslich anormale Ereignisse enthält, bewirkt dies eine Abweichung der statistischen Kenngrösse von dem Sollwert, die der Vergleicher (8) anhand der entsprechenden Signale feststellt und durch Abgabe eines Ausgangssignales AS anzeigt. Die beschriebene Einrichtung (1) ist also in der Lage, Folgen von anormalen Ereignissen unter einer Vielzahl von normalen Ereignissen in einem elektrischen Signal IS zu detektieren.

Den jeweiligen Bedürfnissen entsprechend kann der Vergleicher (8) so aufgebaut sein, dass er ein Ausgangssignal AS erzeugt, wenn das der ermittelten statistischen Kenngrösse entsprechende Signal einen entsprechenden Sollwert übersteigt, unterschreitet oder einen fensterartigen Sollwertbereich verlässt.

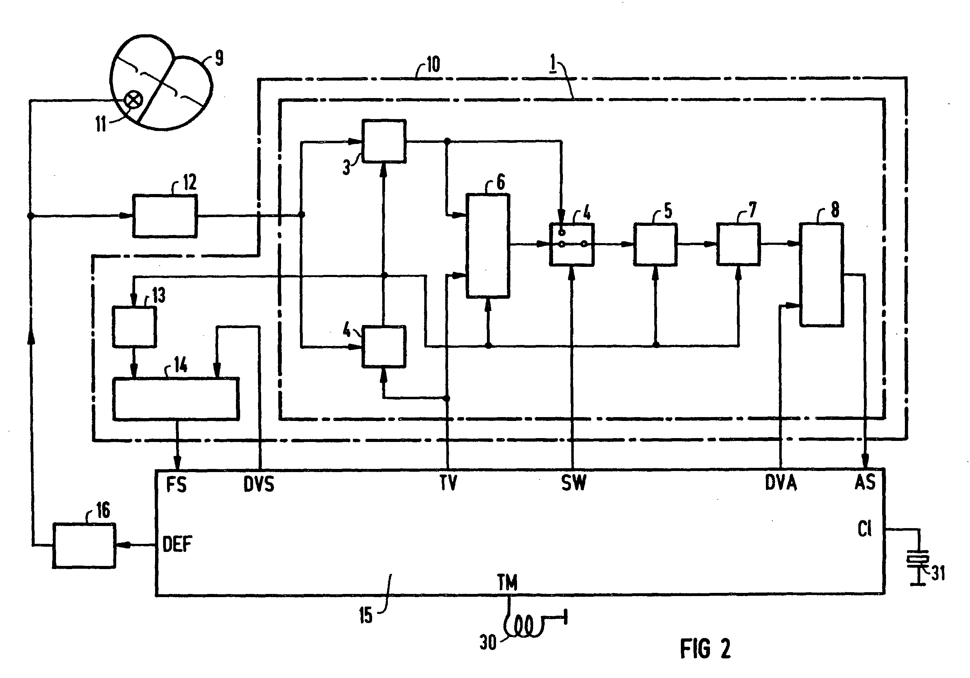

In FIG 2 ist ein Gerät zur selbsttätigen Defibrillation eines eine Funktionsstörung aufweisenden Herzens (9) dargestellt. Derartige Geräte, die ähnlich wie Herzschrittmacher auch in den Körper eines Lebewesens implantiert werden können, falls sie entsprechend miniaturisiert und in einem geeigneten Gehäuse aufgenommen sind, dienen dazu, einen als Herzflimmern bzw. Fibrillation bezeichneten Zustand eines Herzens zu beenden, indem sie das Herz mit Stromstössen, sogenannten Defibrillationsimpulsen, beaufschlagen. Damit ein derartiges Gerät selbsttätig arbeiten kann, muss es zunächst in der Lage sein, das Auftreten von Fibrillation sicher zu detektieren. Hierzu dient bei dem Gerät gemäß FIG 2 eine insgesamt mit (10) bezeichnete Fibrillations-Detektorschaltung, der das Depolarisationssignal des Herzens (9) zugeführt ist. Das Depolarisationssignal wird mittels einer in ein Ventrikel des Herzens (9) eingepflanzte Elektrode (11) abgenommen und durchläuft eine Signalaufbereitungsschaltung (12), in der es verstärkt und erforderlichenfalls gefiltert wird, bevor es zu der Fibrillations-Detektorschaltung (10) gelangt. Die Fibrillations-Detektorschaltung (10) enthält zum einen eine Einrichtung (1) gem. FIG 1, die hier dazu dient, in dem Depolarisationssignal des Herzens (9) eine Folge von anormalen, auf eine Fibrillation hindeutenden Ereignissen zu detektieren. Diesbezügliche Einzelheiten werden noch erläutert. Es sei jedoch an dieser Stelle bereits darauf hingewiesen, dass der Speicher 5 eine Kapazität aufweist, die ausreicht, um die Maximalwerte bzw. diesen entsprechenden Daten für die wenigsten 20, bzw. typisch 50 zuletzt aufgetretenen Ereignisse zu speichern, und dass die Rechenschaltung (7) als Kenngrösse bez. deren statistischer Verteilung die Standardabweichung ermittelt, die mittels des Vergleichers 8 mit einem entsprechenden Sollwert bzw. einem diesem entsprechenden Signal DVA verglichen wird. Ausserdem enthält die Fibrillations-Detektorschaltung (10) eine Mess-Schaltung (13), der die von der Einrichtung (1) stammenden Detektionssignale DS zugeführt sind und die die Zeitintervalle zwischen aufeinanderfolgenden Detektorsignale DS, also eine der Herzschlagfrequenz entsprechende Grösse misst. Das Ausgangssignal der MessSchaltung (13) gelangt zu einem Vergleicher (14) und wird hier mit einem einem Sollwert entsprechenden Signal DVS verglichen, dem eine bestimmte Herzschlagfrequenz entspricht, bei deren Überschreiten möglicherweise eine Fibrillation vorliegt. Im Falle des Überschreitens liefert der Vergleicher (14) ein Ausgangssignal FS, das ebenso wie das Ausgangssignal AS des Vergleichers (8) einer mit der Fibrillations-Detektorschaltung (10) verbundenen Steuerlogik (15) zugeführt ist.

Liefert der Vergleicher (8) ein Ausgangssignal AS, das besagt, dass eine Folge von anormalen, auf eine Fibrillation hindeutenden Ereignissen in dem Depolarisationssignal des Herzens (9) detektiert wurde, und liefert gleichzeitig der Vergleicher (14) ein Signal FS, das besagt, dass die Herzschlagfrequenz in einem Bereich liegt, der für eine Fibrillation typisch ist, betätigt die Steuerlogik (15) mittels eines Signales DEF über einen entsprechend bezeichneten Ausgang eine Generatoreinrichtung (16) derart, dass diese einen oder mehrere Defibrillationsimpulse abgibt, die dem Herzen (9) über die gleiche Elektrode (11), die auch zur Erfassung des Depolarisationssignales dient, zugeführt werden. Es versteht sich, dass zur Beaufschlagung des Herzens (9) mit Defibrillationsimpulsen auch eine gesonderte Elektrode vorhanden sein kann.

Wie aus der FIG 2 anhand von Ausgängen der Steuerlogik (15) ersichtlich ist, die die entsprechenden Bezeichnungen tragen, werden der Fibrillations-Detektorschaltung (10) die Signale TV, DVA, DVS und SW, deren Bedeutung bereits erläutert wurde, von der Steuerlogik (15) zugeführt.

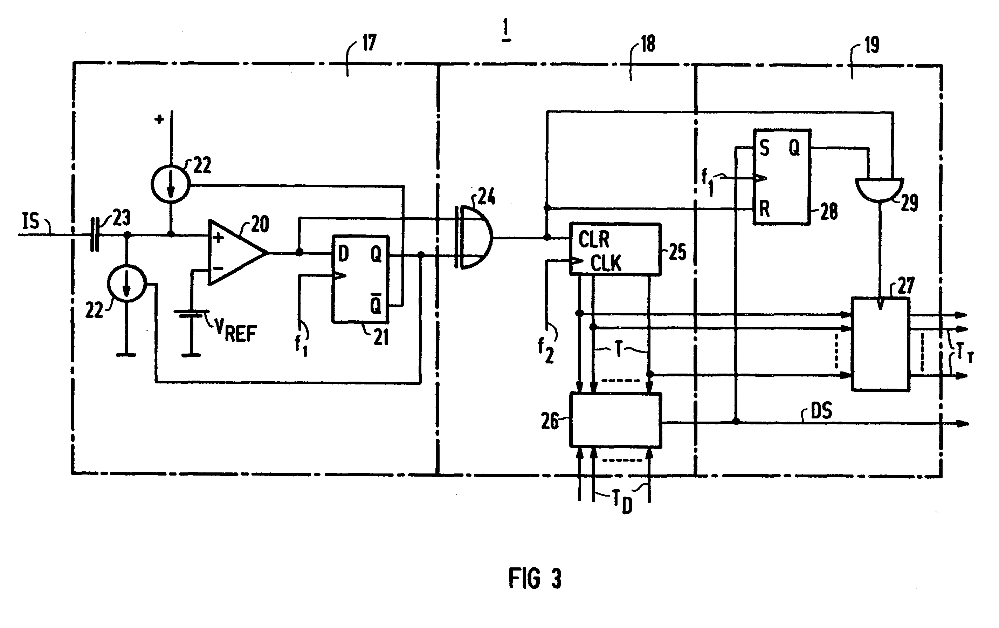

Die in FIG 3 dargestellte detaillierte Schaltung, die als Teil der Einrichtung (1) in der Fibrillations-Detektorschaltung (10) des Gerätes gem. FIG 2 Verwendung findet, führt die Funktionen der Detektorschaltung (2) und des Spitzenwertmessers (3) aus. Dabei ist die Schaltung gem. FIG 3 als Digitalschaltung ausgeführt, was im Hinblick auf das Zusammenwirken mit der Steuerlogik (15) von Vorteil ist. Eine der in FIG 3 dargestellten Schaltung entsprechende Schaltung lässt sich jedoch auch ohne weiteres als Analog-Schaltung verwirklichen, falls dies wünschenswert ist. Die Schaltung gem. FIG 3 umfasst einen Delta-Modulator (17), eine Zählereinheit (18) und eine Registereinheit (19).

Der Delta-Modulator (17) weist einen Komparator (20), ein D-Flip-Flop (21), Stromquellen (22) und einen Integrations-Kondensator (23) auf. Der invertierende Eingang des Komparators (20) ist mit einer Referenzspannung V_{REF} verbunden. Das D-Flip-Flop (21) wird mit einer Frequenz f₁ von beispielsweise 16 kHz getaktet. Die Zählereinheit (18) weist ein EXKLUSIV-ODER-Gatter (24), einen mit einer Frequenz f₂ von beispielsweise 8 kHz getakteten Zähler (25) und einen Vergleicher (26) auf. Der Vergleicher (26) vergleicht den Ausgangswert T des Zählers (25) mit einem Sollwert TV, der einer bestimmten Zeitdauer t_{D} entspricht. Für den Fall, dass der Ausgangswert des Zählers (25) den Schwellwert TV übersteigt, wird ein Detektionssignal DS erzeugt. Die Registereinheit (19) enthält ein Register (27), das über ein RS-Flip-Flop (28) und ein UND-Gatter (29) getaktet wird, wobei das RS-Flip-Flop (28) seinerseits mit einer Frequenz f₁ getaktet ist. Das Register (27) dient dazu, den maximalen Ausgangswert des Zählers (25) zu speichern, den dieser jeweils im Anschluss an die Erzeugung eines Detektionssignals DS erreicht.

Solange die Steilheit des der Schaltung gem. FIG 3 zugeführten Depolarisationssignales IS weder den Wert + I/C überschreitet noch den Wert -I/C unterschreitet, wobei + I bzw. -I dem durch die Stromquellen (22) fliessenden Strom, der einstellbar sein kann, und C der Kapazität des Integrations-Kondensators (23) entspricht, schaltet das D-Flip-Flop (21) ständig um, was zur Folge hat, dass der Zähler (25) in der Zählereinheit (18) über das EXKLUSIV-ODER-Gatter (24) ständig auf Null zurückgesetzt wird. Überschreitet bzw. unterschreitet die Steilheit des Depolarisationssignales IS den Wert + I/C oder -I/C behält der Ausgang des Komparators (20) seinen Zustand bei, solange die Amplitude des Depolarisationssignals IS den Wert (+ I/C)t bzw. (-I/C)t über- bzw. unterschreitet, wobei t die Zeit ist, die seit dem über-bzw. unterschreiten der Steilheit + I/C bzw. -I/C verstrichen ist. Solange der Ausgangszustand des Komparators (20) konstant bleibt, ändert auch das D-Flip-Flop (21) seinen Zustand nicht, so dass der Zähler (25) von dem EXKLUSIV-ODER-Gatter (24) nicht zurückgesetzt wird. Demzufolge beginnt der ein Mass für die Zeit t darstellende Ausgangswert T des Zählers (25) zu steigen. Erreicht der Ausgangswert T des Zählers (25) einen Wert T_{D}, der einer Mindestzeitdauer t_{D} entspricht, gibt der Vergleicher (26), der den Ausgangswert des Zählers (25) mit dem Wert T_{D}, der dem Signal TB gemäss den FIG 1 und 2 entspricht, vergleicht, ein Detektionssignal DS ab, das darauf hinweist, dass ein Ereignis detektiert wurde. Im Anschluss daran zählt der Zähler (25) weiter, bis er von dem EXKLUSIV-ODER-Gatter (24) auf Null zurückgesetzt wird. Dies geschieht dann, wenn die Amplitude des Depolarisationssignals IS den Wert (+I/C)t bzw. (-I/C)t über- bzw. unterschreitet, da dann der Komparator (20) und mit diesem das D-Flip-Flop (21) seinen Ausgangszustand ändert. Die bis zu diesem Zeitpunkt seit dem Über- bzw. Unterschreiten der Steilheit +I/C bzw. -I/C verstrichene Gesamtzeitdauer t_{T} entspricht der Ausgangswert T_{T} des Zählers (25), der in das Register (27) der Registereinheit (19) übertragen wird und von dort in den Speicher (5) (siehe FIG 1 und 2) übertragen wird. Dass die Übertragung des Ausgangswertes des Zählers (25) in das Register (27) erfolgt, bevor der Zähler (25) auf Null zurückgestellt wird, wird durch das RS-Flip-Flop (28) in Verbindung mit dem UND-Gatter (18) erreicht. Das UND-Gatter (18) gibt nämlich einen Taktimpuls an das Register (27), wenn nach der Detektion eines Ereignisses zunächst das RS-Flip-Flop (28), dessen Ausgang mit dem einen Eingang des UND-Gatters (29) verbunden ist, gesetzt wurde und dann ein von dem EXKLUSIV-ODER-Gatter (24) stammender Impuls an dem anderen Eingang des UND-Gatters (29) eintrifft.

Sobald die Steilheit des Depolarisationssignals IS wieder den Wert + I/C bzw. -I/C über- bzw. unterschreitet, wiederholt sich der beschriebene Vorgang.

Es wird deutlich, dass es sich bei der Schaltung gem. FIG 3 bei dem Signalparameter, bezüglich dessen das Depolarisationssignal IS mit einem Schwellwert verglichen und bezüglich dessen der Maximalwert gemessen wird, um die Zeitdauer handelt, für die die Amplitude des Depolarisationssignals IS einen Wert über- bzw. unterschreitet, der sich aus dem Produkt einer definierten Steilheit + I/C bzw. -I/C und derjenigen Zeitdauer t ergibt, die seit dem Zeitpunkt verstrichen ist, zu dem die Steilheit des Depolarisationssignals IS die definierte Steilheit +I/C bzw. -I/C erreicht. Damit eine Wellenform in dem Depolarisationssignal IS als Ereignis detektiert wird, muss sie also eine bestimmte Steilheit + I/C bzw. -I/C über- bzw. unterschreiten, eine bestimmte Mindestzeitdauer t_{D} erreichen und eine bestimmte Mindestamplitude (+I/C)t bzw. (-I/C)t aufweisen.

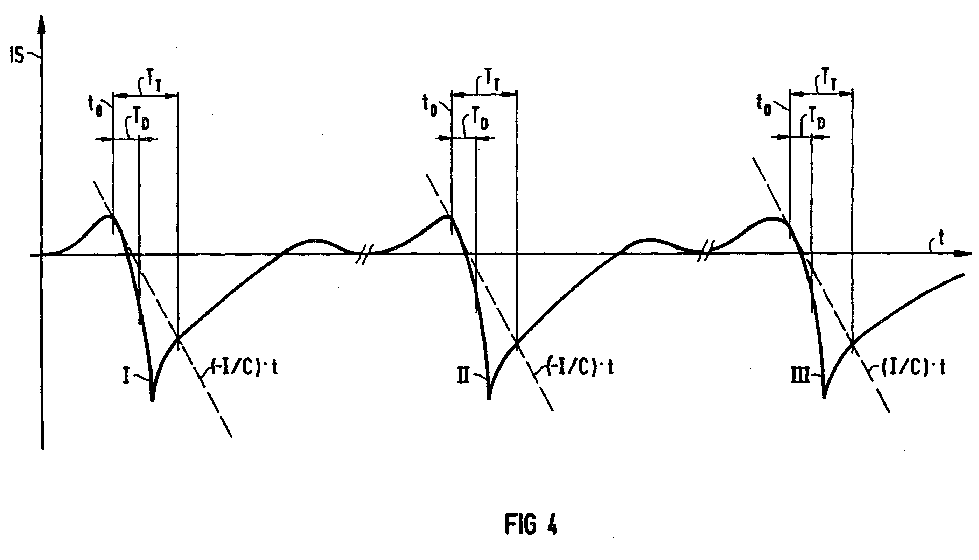

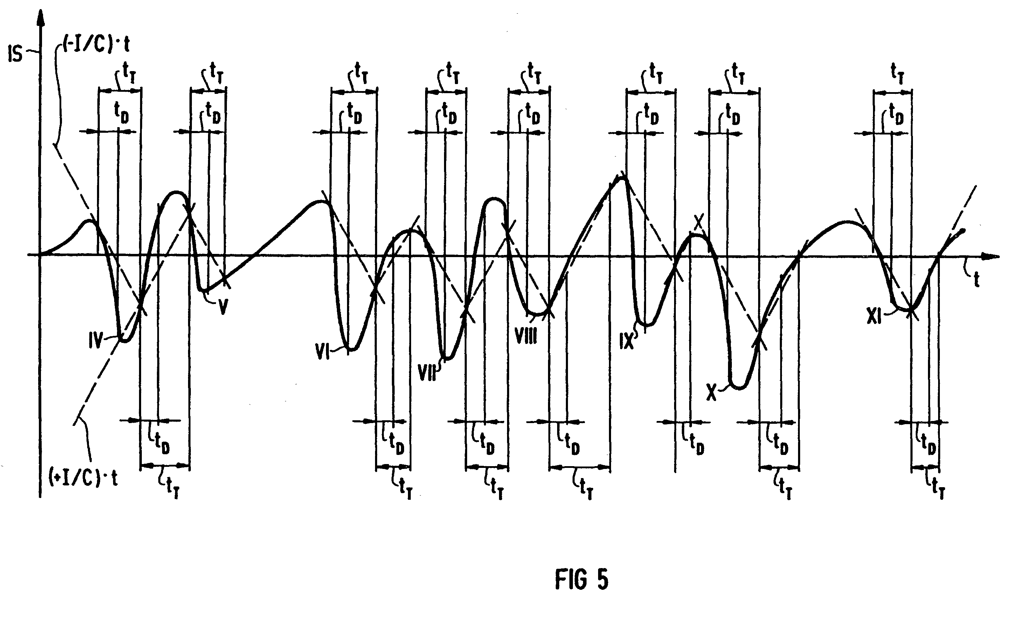

Anhand der FIG 4 und 5 wird die Arbeitsweise der Schaltung gem. FIG 3 und deren Fähigkeit, auf eine Fibrillation des Herzens hindeutende Folgen von Ereignissen zu detektieren, nochmals deutlicher.

In der FIG 4 ist das Depolarisationssignal eines im Sinus-Rhythmus, also im normalen Rhythmus, arbeitenden Herzens dargestellt, wie es am Ausgang der Signalaufbereitungsschaltung (12) der Einrichtung gem. FIG 2 vorliegt. Der dargestellte Ausschnitt des Depolarisationssignals enthält 3 Ereignisse, die mit I bis III bezeichnet sind. Die Einrichtung 1 überprüft dem Takt der Frequenz f₁ folgend fortlaufend, ob das Depolarisationssignal eine Steilheit aufweist, die den Wert + I/C über-bzw. den Wert -I/C unterschreitet. Solange dies nicht der Fall ist, wird der Zähler (25) laufend zurückgesetzt. In FIG 4 weist das Ereignis I zum Zeitpunkt t₀ erstmals eine Steilheit auf, die den Wert -I/C unterschreitet. Ausgehend von diesem Zeitpunkt misst der Zähler (25) die Zeitdauer t_{T} bis zu demjenigen Zeitpunkt, zu dem die Amplitude des Depolarisationssignals den Wert (-I/C)t überschreitet. Der Verlauf der Geraden (-I/C)t ist in FIG 4 eingetragen, wobei diese Gerade das Depolarisationssignal zum Zeitpunkt t₀ tangiert und nach Ablauf der Gesamtzeitdauer t_{T} schneidet. Da im Falle des Ereignisses I die Gesamtzeitdauer t_{T} eine Mindestzeitdauer t_{D}, z.B. 6 ms, überschreitet, erzeugt die Einrichtung (1) nach Ablauf der Mindestzeit t_{D} ein Detektionssignal DS. Daten, die der Gesamtzeitdauer t_{T} entsprechen, werden in den Speicher (5) übertragen. Es kann sich dabei um Daten handeln, die entweder der Gesamtzeitdauer t_{T} selbst oder z.B. der Differenz aus der Gesamtzeitdauer t_{T} und der Mindestzeitdauer t_{D} entsprechen. Aus der FIG 4 wird deutlich, dass die für die zuletzt eingetretenen Ereignisse I bis III bezüglich der genannten Daten ermittelte Standardabweichung relativ geringfügig schwanken wird, da die Gesamtzeitdauer t_{T} für die einzelnen Ereignisse I bis III nur geringfügig schwankt.

Dies trifft nicht zu, wenn eine Fibrillation des Herzens, das entsprechende Depolarisationssignal ist in FIG 5 dargestellt, vorliegt. In diesem Falle schwanken die für die einzelnen Ereignisse ermittelten Gesamtzeitdauern t_{T} erheblich. Im Gegensatz zu der FIG 4, die das Depolarisationssignal eines nach dem Sinus-Rhythmus arbeitenden Herzens zeigt, sind im Falle des für Fibrillation typischen Depolarisationssignals gem. FIG 5 auch die ansteigenden Flanken der Ereignisse so steil, dass eine Detektion-möglich ist. Dies bedeutet, dass für die Ereignisse IV bis XI teilweise zwei Detektionssignale DS erzeugt werden, da die Mindestzeitdauer t_{D} sowohl im Falle der abfallenden als auch der ansteigenden Flanke eines Ereignisses überschritten wird.

Anhand der FIG 4 und 5 sowie der entsprechenden Erläuterungen wird deutlich, dass die Standardabweichung S im Falle von Sinus-Rhythmus wesentlich geringer als im Falle von Fibrillation ist. Es wird somit deutlich, dass durch Vergleich der Standardabweichung S mit einem geeigneten Sollwert erkennbar ist, ob ein Depolarisationssignal eine Folge von auf Fibrillation hindeutenden Ereignissen enthält bzw. vollständig aus solchen Ereignissen besteht. Unter zusätzlicher Heranziehung der Herzschlagfrequenz (FIG 2) ist es möglich, die Fibrillation des Herzens eindeutig zu detektieren.

Wie aus FIG 2 anhand des Anschlusses TM der Steuerlogik (15) und der mit diesem verbundenen Sende-/Empfangsspule (30) ersichtlich ist, ist die Steuerlogik (15) derart ausgebildet, dass das Gerät gem. FIG 2 mit einem nicht dargestellten externen Gerät bidirektional telemetrisch kommunizieren kann. Demgemäss besteht die Möglichkeit, die in der Steuerlogik (15) gespeicherten Daten bezüglich der Signale DVA, DVS, TV zu überprüfen und bei Bedarf zu verändern. Die gleiche Möglichkeit besteht auch bezüglich der Einstellbarkeit der Stromquellen (22) und einem weiteren, zusätzlich möglichen Detektionskriterium, nämlich der Polaritätswechsel I/C, + I/C oder -I/C für die Steilheit.

Die Frequenzen f₁, f₂, werden übrigens in allgemein bekannter und daher nicht dargestellter Weise aus der Schwingungsfrequenz eines über den Anschluss Cl mit der Steuerlogik (15) verbundenen Quarzes (31) abgeleitet.

## Patentansprüche

1. Einrichtung zum Detektieren einer Folge von anormalen funktionsstörungsbedingten Ereignissen unter einer Vielzahl von normalen Ereignissen in einem elektrophysiologischen Signal (IS) eines eine Funktionsstörung aufweisenden Organs, welche Einrichtung aufweist:
a) Mittel (2;17,18,19) zum Vergleichen des Signals (IS) hinsichtlich eines Signalparameters (t;T) mit einem definierten Schwellwert (t_{D};T_{D}), den das Signal (IS) sowohl beim Auftreten eines normalen als auch eines anormalen Ereignisses überschreitet,
b) Mittel (3;17,18,19) zur Messung des Maximalwertes (t_{T};T_{T}) des Signalparameters (t;T) des jeweiligen Ereignisses, **gekennzeichnet durch**
c) Mittel (5,7) zum Ermitteln einer der statistischen Verteilung der für das jeweils zuletzt aufgetretene Ereignis und eine definierte Anzahl von aufeinanderfolgend unmittelbar vor diesem aufgetretenen Ereignissen gemessenen Maximalwerten (t_{T};T_{T}) entsprechenden Kenngröße (S), und
d) Mittel (8) zum Vergleichen der ermittelten Kenngröße (S) mit einem definierten Sollwert (DVA), welche bei einer Abweichung zwischen ermittelter Kenngröße (S) und Sollwert (DVA) ein Signal (AS) erzeugen.

2. Einrichtung nach Anspruch 1, **dadurch gekennzeichnet, daß** die Mittel (5,7) zum Ermitteln einer der statistischen Verteilung der gemessenen Maximalwerte (t_{T};T_{T}) entsprechenden Kenngröße (S) die Standardabweichung ermitteln.

3. Einrichtung nach Anspruch 1 oder 2, **dadurch gekennzeichnet, daß** Mittel (4,6)zur Bestimmung der Differenz zwischen dem jeweils für ein Ereignis gemessenen Maximalwert (t_{T};T_{T}) und dem Schwellwert (t_{D};T_{D}) des Signalparameters (t;T) vorgesehen sind, wobei den Mitteln (7) zum Ermitteln einer der statistischen Verteilung der gemessenen Maximalwerte (t_{T};T_{T}) entsprechenden Kenngröße bezüglich der einzelnen Ereignisse jeweils die Differenz zur Ermittlung der Größe (S) zugeführt ist.

4. Einrichtung nach Anspruch 1, wobei das Signal das Depolarisationssignal (IS) eines eine Funktionsstörung aufweisenden Herzens (9) ist und wobei die Folge von anormalen Ereignissen auf eine Fibrillation hindeutet, und welche Einrichtung aufweist:
a) Mittel (11,12) zum Erfassen des Depolarisationssignals(IS) des Herzens (9),
b) Mittel (2,17,18,19) zum Vergleichen des Depolarisationssignals (IS) hinsichtlich derjenigen, als Signalparameter zu verstehenden Zeitdauer (t;T), für die seine Amplitude jeweils beginnend von einem Zeitpunkt (t₀), zu dem die Steilheit des Depolarisationssignals (IS) eine definierte Steilheit (+ I/C,-IC) erreicht, mindestens dem Produkt aus definierter Steilheit (+ I/C,-I/C) und verstrichener Zeit entspricht (t;T), mit einer als Schwellwert zu verstehenden Mindestzeitdauer (t_{D};T_{D}), die das Depolarisationssignal (IS) sowohl beim Auftreten eines normalen als auch eines auf eine Fibrillation hindeutenden Ereignisses überschreitet,
c) Mittel (3;17,18,19) zum Messen der, als Maximalwert zu verstehenden, Gesamtzeitdauer (t_{T}:T_{T}) für die die Amplitude des Depolarisationssignals (IS) während des jeweiligen Ereignisses mindestens dem Produkt aus definierter Steilheit (+ I/C,-I/C) und verstrichener Zeit (t;T) entspricht,
d) Mittel (5.7) zum Ermitteln einer der als statistische Verteilung zu verstehenden Standardabweichung, der für das jeweils zuletzt aufgetretene Ereignis und eine definierte Anzahl von aufeinanderfolgend unmittelbar vor diesem aufgetretenen Ereignissen gemessenen Gesamtzeitdauern (t_{T};T_{T}) entsprechenden statistischen Kenngröße (S), und
e) Mittel (8) zum Vergleichen der ermittelten statistischen Kenngröße (S) mit einem Sollwert (DVA) der Kenngröße (S), welche beim Überschreiten des Sollwertes (DVA) ein Signal (AS) erzeugen.

5. Einrichtung zum Detektieren der Fibrillation eines eine Funktionsstörung aufweisenden Herzens, welche aufweist:
a) eine Einrichtung nach Anspruch 4, zum Detektieren einer Folge von auf eine Defibrillation hindeutenden Ereignissen in dem Depolarisationssignal (IS) des Herzens (9),
b) Mittel (13,14) zum Messen der Herzschlagfrequenz des Herzens (9) und Vergleichen der gemessenen Herzschlagfrequenz mit einem definierten Grenzwert (DVS), und
c) Mittel zum Erzeugen eines Signales (DEF), wenn sowohl die der Standardabweichung entsprechende Kenngröße (S) den Sollwert (DVA) als auch die Herzschlagfrequenz den Grenzwert (DVS) überschreiten.

6. Einrichtung zur selbsttätigen Defibrillation eines eine Funktionsstörung aufweisenden Herzens, welche aufweist:
a) eine Einrichtung nach Anspruch 5 zum Detektieren der Defibrillation des Herzens (9),
b) Mittel (16,11) zur Beaufschlagung des Herzens (9) mit Defibrillationsimpulsen, die mit den Mitteln (15) zum Erzeugen eines Signals (DEF) verbunden sind und bei Vorliegen eines Signales (DEF) das Herz (9) mit wenigstens einem Defibrillationsimpuls beaufschlagen.

7. Einrichtung nach einem der Ansprüche 4 bis 6, **dadurch gekennzeichnet, daß** Mittel (4,6) zur Bestimmung der Differenz zwischen der jeweils für ein Ereignis gemessenen Gesamtzeitdauer (t_{T};T_{T}) und der Mindestzeitdauer (t_{D};T_{D}) vorgesehen sind, wobei den Mitteln (7) zum Ermitteln einer der statistischen Verteilung der gemessenen Maximalwerte (t_{T};T_{T}) entsprechenden Kenngröße (S) bezüglich der einzelnen Ereignisse jeweils die Differenzen zur Ermittlung der Kenngröße (S) zugeführt sind.

## Claims

1. Device for detecting a series of abnormal dysfunction-conditioned events among a multiplicity of normal events in an electrophysiological signal (IS) of an organ exhibiting a dysfunction, which device exhibits:
a) means (2; 17, 18, 19) for comparing the signal (IS), with respect to a signal parameter (t; T), with a defined threshold value (t_{D}), T_{D}), which the signal (IS) exceeds upon the occurrence both of a normal and of an abnormal event,
b) means (3; 17, 18, 19) for measuring the maximum value (t_{T}) T_{T}) of the signal parameter (t; T) of the respective event, **characterized by**
c) means (5, 7) for determining a characteristic quantity (S) corresponding to the statistical distribution of the measured maximum values (t_{T}; T_{T}) measured for the event which occurred respectively last and a defined number of events which occurred in succession directly before the latter, and
d) means (8) for comparing the determined characteristic quantity (S) with a defined theoretical value (DVA), which means generate a signal (AS) in the event of a deviation between the determined characteristic quantity (S) and theoretical value (DVA).

2. Device according to Claim 1, **characterized in that** the means (5, 7) for determining a characteristic quantity (S) corresponding to the statistical distribution of the measured maximum values (t_{T}; T_{T}) determine the standard deviation.

3. Device according to Claim 1 or 2, **characterized in that** means (4, 6) for determining the difference between the maximum value (t_{T}; T_{T}) measured in each instance for an event and the threshold value (t_{D}; T_{D}) of the signal parameter (t; T) are provided, the difference for the determination of the quantity (S) being fed, with respect to the individual events in each instance, to the means (7) for determining a characteristic quantity corresponding to the statistical distribution of the measured maximum values (t_{T}; T_{T}).

4. Device according to Claim 1, in which the signal is the depolarization signal (IS) of a heart (9) exhibiting a dysfunction, and in which the series of abnormal events is indicative of a fibrillation, and which device exhibits:
a) means (11, 12) for acquiring the depolarization signal (IS) of the heart (9),
b) means (2; 17, 18, 19) for comparing the depolarization signal (IS), with respect to that time duration (t; T), to be understood as signal parameter, for which its amplitude, in each instance beginning from an instant (t₀) at which the steepness of the depolarization signal (IS) reaches a defined steepness (+I/C,-IC), at least corresponds to the product of defined steepness (+I/C, -I/C) and elapsed time, with a minimum time duration (t_{D}; T_{D}), to be understood as threshold value, which the depolarization signal (IS) exceeds upon the occurrence of both a normal event and an event which is indicative of a fibrillation,
c) means (3; 17, 18, 19) for measuring the total time duration (t_{T}; T_{T}), to be understood as maximum value, for which the amplitude of the depolarization signal (IS), during the respective event, at least corresponds to the product of defined steepness (+I/C,-I/C) and elapsed time (t; T),
d) means (5, 7) for determining a statistical characteristic quantity (S) corresponding to the standard deviation, to be understood as statistical distribution, of the total time durations (t_{T}; T_{T}) measured for the event which occurred respectively last and a defined number of events which occurred in succession directly before the latter, and
e) means (8) for comparing the determined statistical characteristic quantity (S) with a theoretical value (DVA) of the characteristic quantity (S), which means generate a signal (AS) upon the exceeding of the theoretical value (DVA).

5. Device for detecting the fibrillation of a heart exhibiting a dysfunction, which device exhibits:
a) a device according to Claim 4 for detecting a series of events in the depolarization signal (IS) of the heart (9) which are indicative of a defibrillation,
b) means (13, 14) for measuring the heartbeat rate of the heart (9) and comparing the measured heartbeat rate with a defined limiting value (DVS), and
c) means for generating a signal (DEF) when both the characteristic quantity (S) corresponding to the standard deviation exceeds the theoretical value (DVA) and the heartbeat rate exceeds the limiting value (DVS).

6. Device for the automatic defibrillation of a heart exhibiting a dysfunction, which device exhibits:
a) a device according to Claim 5 for detecting the defibrillation of the heart (9),
b) means (16, 11) for acting on the heart (9) with defibrillation pulses which are connected to the means (15) for generating a signal (DEF) and, in the event of the presence of a signal (DEF), act on the heart (9) with at least one defibrillation pulse.

7. Device according to one of Claims 4 to 6, **characterized in that** means (4, 6) for the determination of the difference between the total time duration (t_{T}; T_{T}) measured in each instance for an event and the minimum time duration (t_{D}; T_{D}) are provided, in each instance the differences for the determination of the characteristic quantity (S) being fed, with respect to the individual events, to the means (7) for determining a characteristic quantity (S) corresponding to the statistical distribution of the measured maximum values (t_{T}; T_{T}).

## Revendications

1. Dispositif pour détecter une suite de phénomènes anormaux dûs à un dysfonctionnement, parmi une multitude de phénomènes anormaux dans un signal électrophysiologique (IS) d'un organe présentant un dysfonctionnement, ce dispositif comportant :
a) des moyens (2;17,18,19) pour comparer un paramètre (t;T) du signal (IS), à une valeur de seuil définie (t_{D}; T_{D}), que le signal (IS) dépasse lors de l'apparition aussi bien d'un évènement normal que d'un évènement anormal,
b) des moyens (3;17,18,19) pour mesurer la valeur maximale (t_{T};T_{T}) du paramètre (t;T) du signal du phénomène respectif,
**caractérisé par**
c) des moyens (5,7) pour déterminer une grandeur caractéristique (S), qui correspond à la répartition statistique des valeurs maximales (t_{T},T_{T}) mesurées pour le phénomène apparu respectivement en dernier lieu et pour un nombre défini de phénomènes apparus successivement juste avant qu'apparaisse ce phénomène, et
d) des moyens (8), qui sont destinés à comparer la grandeur caractéristique (S) déterminée à une valeur de consigne définie (DVA) et qui produisent un signal (AS) dans le cas d'un écart entre la grandeur caractéristique (S) déterminée et la valeur de consigne (DVA).

2. Dispositif suivant la revendication 1, **caractérisé par le fait que** les moyens (5,7) servant à déterminer une grandeur caractéristique (S) correspondant à la répartition statistique des valeurs maximales mesurées (t_{T};T_{T}), déterminent l'écart-type.

3. Dispositif suivant la revendication 1 ou 2, **caractérisé par le fait qu'**il est prévu des moyens (4,6) servant à déterminer la différence entre la valeur maximale (t_{T};T_{T}) mesurée respectivement pour un phénomène, et la valeur de seuil (t_{D};T_{D}) du paramètre (t;T) du signal, la différence pour la détermination de la grandeur (S) étant envoyée respectivement aux moyens (7) pour déterminer une grandeur caractéristique correspondant à la répartition statistique des valeurs maximales mesurées (t_{T};T_{T}), en rapport avec les phénomènes individuels.

4. Dispositif suivant la revendication 1, dans lequel le signal est le signal de dépolarisation (IS) du coeur (9) ayant un dysfonctionnement et la suite de phénomènes anormaux indique une fibrillation, lequel dispositif comporte :
a) des moyens (11,12) pour détecter le signal de dépolarisation (IS) du coeur (9),
b) des moyens (2;17,18,19) pour comparer la durée (t;T) devant être considérée en tant que paramètre du signal de dépolarisation (IS), pendant laquelle son amplitude correspond, respectivement à partir d'un instant (t₀), auquel la pente du signal de dépolarisation (IS) atteint une pente définie (+I/C, -I/C), au moins au produit de la pente définie (+I/C, -I/C) par le temps écoulé, à une durée minimale (t_{D};T_{D}) qu'il faut considérer comme une valeur de seuil et que dépasse le signal de dépolarisation (IS) aussi bien lors de l'apparition d'un phénomène normal que lors de l'apparition d'un phénomène indiquant une fibrillation,
c) des moyens (3;17,18,19) pour mesurer la durée totale (t_{T}; T_{T}), devant être considérée comme une valeur maximale et pour laquelle l'amplitude du signal de dépolarisation (IS) correspond, pendant le phénomène respectif, au moins au produit d'une pente définie (+I/C, -I/C) par le temps écoulé (t;T),
d) des moyens (5,7) pour déterminer une grandeur caractéristique statistique (S), qui correspond à l'écart-type à considérer comme une répartition statistique, des durées totales (t_{T};T_{T}) mesurées pour le phénomène apparu respectivement en dernier lieu et pour un nombre défini de phénomènes apparus successivement juste avant que ce phénomène apparaisse, et
e) des moyens (8) pour comparer la grandeur caractéristique statistique déterminée (S) à une valeur de consigne (DVA) de la grandeur caractéristique (S), et qui produisent un signal (AS) lors du dépassement de la valeur de consigne (DVA).

5. Dispositif pour détecter la fibrillation d'un corps présentant un dysfonctionnement, et qui comporte :
a) un dispositif selon la revendication 4 pour détecter une suite de phénomènes indiquant une défibrillation, dans le signal de dépolarisation (IS) du coeur (9),
b) des moyens (13,14) pour mesurer la fréquence des battements du coeur (9) et comparer la fréquence mesurée des battements cardiaques à une valeur limite définie (DVS), et
c) des moyens pour produire un signal (DEF), lorsqu'aussi bien la grandeur caractéristique, qui correspond à l'écart-type dépasse la valeur de consigne (DVA) et la fréquence des battements cardiaques dépasse la valeur limite (DVS).

6. Dispositif de défibrillation automatique du coeur présentant un dysfonctionnement, qui comprend :
a) un dispositif suivant la revendication 5 pour détecter la défibrillation du coeur (9),
b) des moyens (16,11) pour charger le coeur (9) d'impulsions de défibrillation, qui sont reliés aux moyens (15) pour produire un signal (DEF) et, qui, lors de la présence d'un signal (DEF), soumettent le coeur (9) à au moins une impulsion de défibrillation.

7. Dispositif suivant l'une des revendications 4 à 6, **caractérisé par le fait qu'**il est prévu des moyens (4,6) pour déterminer la différence entre la durée totale (t_{T}; T_{T}), mesurée respectivement pour un phénomène, et la durée minimale (t_{D};T_{D}), les différences pour la détermination de la grandeur caractéristique (S) sont envoyées respectivement aux moyens (7) pour déterminer une grandeur caractéristique (S), qui correspond à la répartition statistique des valeurs maximales mesurées (t_{T}; T_{T}), en rapport avec les phénomènes individuels.
